# EUROPÄISCHE PATENTSCHRIFT

(11) **EP 1 132 040 B1**
(45) Veröffentlichungstag und Bekanntmachung des Hinweises auf die Patenterteilung: **09.06.2004**
(21) Anmeldenummer: 01105296.6
(22) Anmeldetag: 05.03.2001
(51) Int. Cl.: A61B 1/005, A61B 1/018, B25J 1/02, B25J 1/00

(54) **Endoskopschaft mit beweglichem distalen Ende**
Endoscope shaft with mobile distal end
Tige endoscopique à extrémité distale mobile

(30) Priorität: 06.03.2000 DE 10010932
(43) Veröffentlichungstag der Anmeldung: 12.09.2001
(73) Patentinhaber: STM Medizintechnik Starnberg GmbH, 86947 Schwabhausen/Weil (DE)
(72) Erfinder: Pauker, Fritz, 86316 Friedberg (DE)
(74) Vertreter: Leson, Thomas Johannes Alois, Dipl.-Ing.

(56) Entgegenhaltungen:
- DE-A- 4 133 605
- US-A- 4 580 551
- US-A- 5 337 732

## Beschreibung

Die vorliegende Erfindung betrifft einen Endoskopschaft gemäß dem Oberbegriff des Patentanspruchs 1.

Endoskope sind Geräte, insbesondere zur Exploration von Hohlräumen oder röhrenartigen Kanälen des Körpers, insbesondere für medizinische Zwecke. Besonders eingeführt haben sich Endeskope zur Exploration der Speiseröhre, des Magens, des Zwölffingerdarms vom Magen aus, des Darms vom Anus aus, der Harnröhre, der Blase und der Harnleiter. Ein derartiges Endoskop ist an seinem Vorderende mit einer Beleuchtungseinrichtung sowie mit einer Optik zur visuellen Erfassung des davor liegenden Bereichs des zur explorierenden Körperhohlraums oder Körperkanals ausgerüstet. Während bis vor kurzem die vor dem Vorderende des Endoskops erfasste, optische Information normalerweise mittels, einer Faseroptik durch das Endoskop nach hinten zu seinem Bedienungsende übertragen worden ist, stellt jetzt der Einbau eines Kamerachips am vorderen Endoskopende sowie die elektrische Bildübertragung und die Darstellung der gewonnenen optischen Information auf einem Bildschirmmonitor den neuesten Stand der Technik dar.

Endoskope weisen ferner in der Regel einen sog. Arbeitskanal auf, durch den diverse Arbeitsinstrumente eingeführt und bedient werden können. Beispielsweise werden kleine Zangen zur Entnahmen von Gewebeproben, Biopsienadeln, beheizbare Schneiddrähte, kleine Scheren, Koagulationselektroden oder dergleichen eingeführt, um ggf. operative Maßnahmen an beschädigtem Gewebe durchzuführen. Schließlich sind in der Regel ein Fluidkanal für Spülflüssigkeit und Bedienungstdrähte zum Abwinkeln des Endoskopvorderendes in mehrere Richtungen vorhanden. Diese Bedienungsdrähte werden wiederum durch einzelne Kanäle innerhalb des Endoskopschafts zu dessen vorderem bzw. distalem Ende geführt, um dieses bis zu 160° in Gegenrichtung des Endoskopschafts dreidimensional zu biegen.

Hierbei ergeben sich nunmehr wesentliche Probleme insbesondere bezüglich des taktilen Gefühls, welches auf den Bediener während des Biegevorgangs des distalen Endes vermittelt wird. Es besteht daher die Gefahr, dass beispielsweise bei der Exploration des Darms, die Darmwand während des Biegevorgangs des distalen Endes beschädigt wird. Darüber hinaus muss eine exakte Positionierbarkeit des distalen Endes an der zu explorierenden und ggf. zu behandelnden Stelle möglich sein, was eine ausreichende Biegefähigkeit bei gleichzeitiger Steifigkeit nach Erreichen der vorbestimmten Biegeposition erforderlich macht.

Im Oberbegriff des neuen Hauptanspruchs wird indessen von einem Endoskopschaft ausgegangen, wie er aus der US-A-4 580 551 bekannt ist. Der dort gezeigte Endoskopschaft hat einen flexiblen Schlauchkörper, an dessen distalem Ende ein abkrümmbares Endstück ausgebildet ist. Das distale Endstück lässt sich mittels einer Betätigungsvorrichtung abwinkeln.

Schließlich sei noch auf die DE 41 33 605 A1 verwiesen. Diese Druckschrift betrifft einen flexiblen Roboterarm, welcher eine Vielzahl von ringförmigen Balken oder Schlauchelementen aufweist, die jeweils aus zwei pneumatisch getrennten Schlauchteilen bestehen. Die Schlauchelemente sind dabei so angeordnet, dass eine Ausrichtung der Schlauchteile auf 0 Uhr, 3 Uhr, 6 Uhr und 9 Uhr erfolgt. Die Schlauchelemente sind dabei durch Pneumatikleitungen derart miteinander verbunden, dass durch eine entsprechende Durckbeaufschlagung ein Abwinkeln des Roboterarms erfolgt. Darüber hinaus sind die Schlauchelemente durch Scharniere miteinander gelenkig verbunden.

Angesichts dieses Stands der Technik ist es die Aufgabe der Erfindung, den distalen Endabschnitt eines Endoskopschafts derart weiterzubilden, dass eine ausreichende Biegefähigkeit sowie eine ausreichende Steifigkeit mit möglichst unkompliziertem Aufbau erzielbar ist.

Diese Aufgabe wird durch einen Endoskopschaft mit den Merkmalen des neuen Patentanspruchs 1 gelöst.

Der distale Endabschnitt des erfindungsgemäßen Endoskops besteht folglich aus einer Mehrzahl von längs neben- bzw. übereinander geschichteter balgförmiger Körper bzw. Schwellkörper zu bilden, von denen jeweils zwei diamterisch zueinanderliegend eine Körperschicht ausbilden und von denen zwei längs, unmittelbar benachbarte Körperpaare 90° zueinander phasenverschoben sind. Hierdurch ergibt sich eine Konstruktion, in welcher die einzelnen Schwellkörper in Längsrichtung gesehen abwechselnd auf null und sechs Uhr gemäß der einen Schicht und auf drei und neun Uhr gemäß der benachbarten Schicht zu liegen kommen. Auf diese Weise ist es möglich, durch entsprechende Betätigung der in der gleichen Winkelposition sich befindlichen Balge bei deren Aufblähen oder Zusammenziehen eine Abkrümmung des distalen Endes in die beabsichtigte Richtung zu erzielen, wobei mit Erreichen der vorbestimmten Abwinklungsposition des distalen Endes die Schwellkörper in dieser Stellung quasi eingefroren werden und damit die Haltung des distalen Endes fixiert wird.

Die Erfindung besteht nunmehr darin, die mechanische Kopplung zwischen zwei axial beabstandeten, jedoch auf der gleichen Winkelposition sich befindenden Schwellkörper gleichzeitig als die Hydraulik- bzw. Pneumatikverbindung auszubilden. Dies geschieht dadurch, dass die mechanische Kopplung mittels eines Leitungsstücks erfolgt, welches zwischen jeweils zwei diametrisch zueinander liegenden Schwellkörpern zueinander verläuft, welche die Schicht zwischen den zu verbindenden Schwellkörpern bildet, wobei das Leitungsstück fest an den zu koppelnden Schwellkörpern fixiert ist. Auf diese Weise lässt sich eine vereinfachte Konstruktion ermöglichen.

Als besonders vorteilhaft hat sich dabei ergeben, die Schwellkörper als hydraulisch oder pneumatisch betätigbare Balge auszubilden, wodurch eine einfache und kostengünstige Betätigungsart beispielsweise durch eine entsprechend ausgebildete Druckmittelquelle beispielsweise in Form einer Handpumpe ermöglicht wird, die zudem ein ausreichendes taktiles Gefühl auf den Bediener vermittelt.

Weitere vorteilhafte Ausgestaltungen der Erfindung sind Gegenstand der übrigen Unteransprüche.

Die Erfindung wird nachstehend anhand eines bevorzugten Ausführungsbeispiels unter Bezugnahme auf die begleitenden Zeichnungen näher erläutert.

Figur 1 zeigt eine Prinzipdarstellung eines distalen Endabschnitts eines Endoskopschafts gemäß einem bevorzugten Ausführungsbeispiel der Erfindung in Querschnittsdarstellung. Figur 2 zeigt den distalen Endabschnitt in Draufsicht als Prinzipdarstellung,

Figur 3a bis 3d zeigen den Aufbau eines Schwellkörpers und

Figur 4 zeigt die Aneinanderreihung einer Mehrzahl von Schwellkörpern in Perspektivenansicht unter Ausbildung des distalen Endes gemäß dem bevorzugten Ausführungsbeispiel.

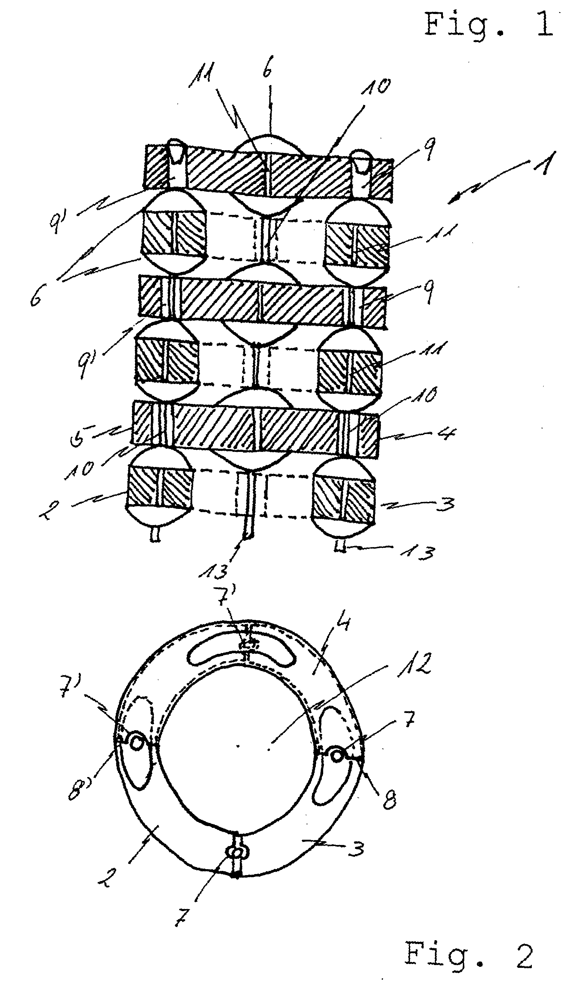

In Figur 1 ist der bewegliche distale Endabschnitt eines Endoskopschafts gemäß dem bevorzugten Ausführungsbeispiel der Erfindung als Prinzipskizze dargestellt.

Wie aus der Figur 1 zu entnehmen ist, besteht der bewegliche distale Endabschnitt 1 des erfindungsgemäßen Endoskopschafts aus einer Mehrzahl von längs neben- bzw. übereinander geschichteter Körper 2, 3, 4, wobei jede Schicht aus jeweils zwei diametrisch zueinander liegender Körpern aufgebaut ist.

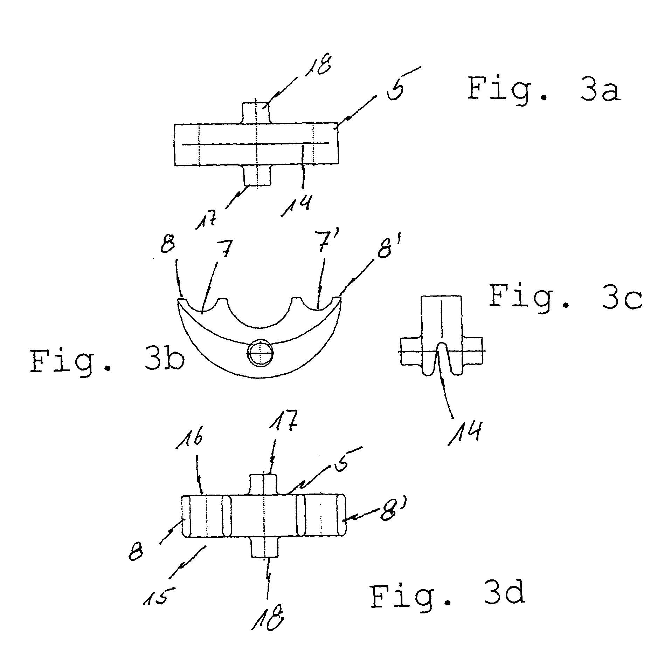

In Figur 2 ist die Draufsicht des beweglichen distalen Endabschnitts 1 als Prinzipdarstellung abgebildet. Jeder Körper 2, 3, 4 besteht demzufolge aus einem halbkreisförmigen, scheibenartigen Ringelement 5, das in Umfangsrichtung gesehen in seinem Mittenabschnitt Schwellkörper 6 ausbildet. An den zwei jeweiligen Endseiten der halbkreisförmigen Ringscheibe 5 sind in Dickenrichtung der Scheibe 5 sich erstreckende Einkerbungen7, 7' vorzugsweise halbkreisförmig ausgebildet. Die halbkreisförmigen Ringscheiben 5 sind dabei in jeder Schicht an ihren beiden Endseiten 8, 8' aneinander gelegt, derart, dass die hierbei sich gegenüberliegenden Einkerbungen 7,7; 7', 7' an den jeweiligen Endseiten zwei diametrisch sich gegenüberliegende Durchgangsöffnungen 9, 9' definieren.

Wie ferner aus der Figur 1 zu entnehmen ist, sind die Körperpaare, welche unmittelbar in Längsrichtung des distalen Endabschnitts benachbart liegen, zusätzlich 90° zueinander phasenverschoben. Hierdurch ordnen sich jeweils abwechselnd die Schwellkörper 6 bezüglich der einen Schicht auf drei und neun Uhr und auf der jeweils benachbarten Schicht auf null und sechs Uhr an.

Die Schwellkörper 6 sind gemäß dem vorliegenden Ausführungsbeispiel als dehnbare Balge ausgebildet, welche pneumatisch oder hydraulisch betätigbar sind. Alternativ hierzu ist es natürlich auch möglich die Schwellkörper 6 als piezoelektrische Elemente auszubilden.

Die Schwellkörper 6, vorliegend die aufweitbaren Balge bilden einen Schwenk- bzw. Abkrümmmechanismus des beweglichen distalen Endes. Hierfür sind sämtliche Balge mit gleicher Winkelposition, d.h. die Balge auf Null-Uhr-Position, auf Drei-Uhr-Position, auf Sechs-Uhr-Position und auf Neun-Uhr-Position jeweils miteinander gekoppelt. Diese Kopplung besteht aus einem Leitungsstück 10, welches zwei längsbeabstandete Balge der gleichen Winkelposition sowohl mechanisch als auch hydraulisch bzw. pneumatisch miteinander verbindet, wobei diese Leitungsstücke 10 durch die Durchgangsöffnungen 9, 9'des dazwischen gelagerten Körperpaars jeweils verläuft. Durch die Leitungsstücke 10 wird demzufolge eine hydraulische bzw. pneumatische Fluidverbindung wie auch eine mechanische Kopplung für das Verhindern eines Auseinanderfallens der einzelnen Schichten erzeugt.

Die Balge befinden sich gemäß der Figur 1 prinzipiell an den sich gegenüberliegenden flachen Seiten der halbkreisförmigen Ringscheiben 5 und sind durch Durchgangsbohrungen 11, welche durch die Ringscheiben 5 sich erstrecken, miteinander fluidverbunden.

Wie aus der Figur 2 zu erkennen ist, wird durch die erfindungsgemäße Aneinanderlagerung der halbkreisförmigen Ringscheiben 5 pro Schicht eine zentrale Durchgangsöffnung 12 gebildet, welche sich längs des gesamten beweglichen distalen Endabschnitts 1 erstreckt und einen Arbeitskanal für das Einführen von chirurgischen Instrumenten, Hilfsinstrumenten oder Optiken bildet.

Das Funktionsprinzip des erfindungsgemäßen beweglichen distalen Endabschnitts lässt sich wie folgt zusammenfassen:

Wird in die fluid gekoppelten Balge auf einer ausgewählten Winkelposition ein Druckmedium beispielsweise eine Hydraulikflüssigkeit über die Leitungsstücke 10 eingepumpt, bewirkt dies ein Aufweiten der Balge im wesentlichen in Längsrichtung des distalen Endabschnitts 1, wodurch sich die halbkreisförmigen Ringscheiben 5 im Bereich dieser Winkelposition voneinander beabstanden. Da sämtliche weiteren Balge auf den jeweils anderen Winkelpositionen nicht druckbeaufschlagt und/oder sogar druckentspannt werden, bewirkt dies ein Abkippen jeder aus zwei Ringscheiben 5 bestehenden Schicht, wodurch sich der distale Endabschnitt über dessen gesamte Längserstreckung allmählich krümmt. Je mehr Hydraulikfluid in die gerade druckbeaufschlagten Balge eingepresst wird, desto größer wird der Krümmungsgrad des Endabschnitts, soweit, dass eine nahezu 160°-Abkrümmung erreicht werden kann.

Eine derartige Abkrümmbewegung in eine Bewegungsrichtung lässt sich natürlich überlagern durch Druckbeaufschlagung von Balgen in einer anderen Winkelposition, beispielsweise in einer hierzu 90° versetzten Winkelposition, wodurch sich eine Art Taummelbewegung des distalen Endabschnitts ergibt. Es ist auch möglich, sämtliche Balge auf allen Winkelpositionen Druck zu beaufschlagen oder zu entspannen um den distalen Endabschnitt im Bereich der Gesamtaufweitungsmöglichkeit aller längs beabstandeten Balge längs zu verschieben oder zusammenzuziehen.

Sobald das distale Ende des beweglichen Endabschnitts eine bestimmte' Krümmungsposition eingenommen hat, wird die Druckbeaufschlagung der jeweilige Balge einer oder mehrerer Winkelpositionen gestoppt, wodurch aufgrund der inkompressiblen Eigenschaft des Hydraulikfluids der distale Endabschnitt in dieser Krümmungsposition fix gehalten wird.

Diese fixe Haltung ist dabei abhängig von der Elastizitiät in Radialrichtung der Balge selbst, wobei bezüglich der Konstruktion jeder halbkreisförmigen Ringscheibe eine gute Elastizität in Längsrichtung jedoch eine möglichst steife Ausgestaltung in Radialrichtung angestrebt wird, wie nachfolgend anhand einer konkreten Konstruktion näher beschrieben wird.

Wie aus der Figur 1 ferner zu entnehmen ist, weisen die halbkreisförmigen Ringscheiben 5 der gemäß Figur 1 untersten Schicht Anschlussstutzen 13 für das Anschließen eines Hydraulikleitungssystems auf. Dieses hydraulische Leitungssystem, welches in den vorliegenden Figuren nicht gezeigt ist, besteht im wesentlichen aus vier Kanälen, die durch den ebenfalls nicht gezeigten Endoskopschaft in darin ausgebildeten Arbeitskanälen geführt und an eine zentrale Hydraulikdruckquelle angeschlossen sind. Als Hydraulikdruckquelle eignet sich dabei vorzugsweise eine handbetätigbare Druckpumpe bestehend aus vier Einzelpumpen, die unabhängig voneinander oder gekoppelt betätigbar sind, derart, dass bei Druckbeaufschlagung der Balge einer Winkelposition der Druck in den Balgen auf der gegenüberliegenden Winkelposition entspannt wird. Durch eine derartige Wechselbeziehung zwischen Druckbeaufschlagung und Druckentspannung auf jeweils gegenüberliegenden Balgreihen lässt sich die Beweglichkeit des distalen Endabschnitts weiter erhöhen und die Positionierfähigkeit verbessern.

Letzteres erlaubt sogar die Exploration beispielsweise der Darmwand im Schließmuskelbereich von der Darmseite aus.

In den Figuren 3a bis 3d ist die Konstruktion einer halbkreisförmigen Ringscheibe 5 in allen Einzelheiten dargestellt. Wie hieraus zu entnehmen ist, besteht die Ringscheibe 5 aus einem Kunststoffkörper mit vorbestimmter Dicke, der einen Hohlraum ausbildet. An der radial äußeren Seite ist die Wandung des Körpers nach innen zumindest einmalig gefaltet, um einen Balg 14 auszubilden.

An den sich gegenseitig gegenüberliegenden Seitenflächen 15, 16 der Ringscheibe 5 sind Anschlussstutzen 17, 18 ausgebildet, welche fluchtend zueinander ausgerichtet sind und in den Hohlraum des Ringkörpers 5 münden. Die Anschlussstutzen 17, 18 können dabei einstückig mit der Ringscheibe 5 ausgebildet oder an diesen angeschweißt sein. Vorzugsweise besteht der Ringkörper 5, aus zwei Schalenhälften, die entlang der radial äußeren und radial inneren Seite in Umfangsrichtung miteinander verschweißt sind.

An den beiden Endflächen 8, 8' der halbkreisförmigen Ringscheibe 5 sind halbkreisförmige Einkerbungen 7, 7' in Dickenrichtung der Ringscheibe 5 ausgebildet.

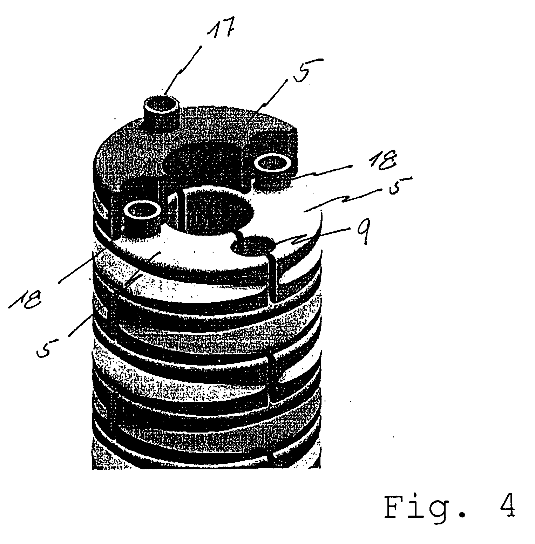

In der Figuren 4 ist der bewegliche distale Endabschnitt konstruktiv dargestellt.

Wie hieraus zu ersehen ist, bilden jeweils zwei der vorstehend beschriebenen Ringscheiben 5, welche an ihren jeweiligen Endflächen 8, 8' aneinandergelegt sind, eine Schicht, wobei die Ringscheibenpaare jeder Schicht, welche unmittelbar benachbart zueinander angeordnet sind, 90° phasenverschoben sind. Zur Fixierung der Ringscheiben 5, welche in Längsrichtung gesehen auf einer Winkelposition liegen, sind die Anschlussstutzen 17, 18, welche durch die Durchgangsöffnungen 9, 9', die durch die endseitig ausgebildeten Einkerbungen 7, 7' in der einen Schicht ausgebildet werden, hindurch sich erstrecken, miteinander verklebt oder verschweißt. Hierdurch wird das vorstehend genannte Leitungsstück 10 sowie die mechanische Verbindung der gekoppelten Ringscheiben 5 in Längsrichtung hergestellt.

Alternativ zu der vorstehend genannten Kunststoffausführung kann natürlich auch Gummi oder Gummilaminat als Material für den distalen Endabschnitt 1 verwendet werden. Der distale Endabschnitt ist dabei auf die endseitige Stirnfläche des Enoskopschafts aufgeschweißt, derart, dass die freien Anschlussstutzen 13 in den zwei untersten Schichten mit den Hydraulikkanälen längs des Hydraulikschafts in Fluidverbindung kommen.

## Patentansprüche

1. Endoskopschaft mit einem beweglichen distalen Endabschnitt (1) sowie einer Betätigungsvorrichtung, die durch den Endoskopschaft hindurch mit dem distalen Endabschnitt (1) für dessen Betätigung wirkverbunden ist,
**dadurch gekennzeichnet, daß**
der distale Endabschnitt (1) aus einer Mehrzahl von längs nebeneinander geschichteter Schwellkörper (5) besteht, von denen jeweils zwei diametrisch zueinander liegend eine Schicht bilden und jeweils zwei längs unmittelbar benachbarte Körperpaare 90° phasenverschoben sind, wobei jeweils alle auf null, auf drei, auf sechs und auf neun Uhr liegenden Schwellkörper (5) miteinander mittels einer mechanischen Kopplung längs gekoppelt sind, die jeweils aus zumindest einem gleichzeitig auch die Hydraulik- oder Pneumatikverbindung bildenden Leitungsstück (10) besteht, welches zwischen jeweils zwei diametrisch zueinander liegenden Schwellkörpern verläuft und fest an den zu koppelnden Schwellkörpern fixiert ist.

2. Endoskopschaft nach Anspruch 1, **dadurch gekennzeichnet, daß**
jeder Balg einen halbkreisförmig gebogenen Grundkörper hat, so daß die zwei Körperpaare jeder Schicht in Zusammenwirkung eine zentrale Durchgangsöffnung (12) bilden.

3. Endoskopschaft nach Anspruch 2, **dadurch gekennzeichnet, daß**
der Grundkörper einen Hohlraum bildet, und an seiner radialen Außenseite gefaltet ist.

4. Endoskopschaft nach Anspruch 3, **dadurch gekennzeichnet, daß**
der Grundkörper an seinen beiden Stirnseiten (15, 16) mit Anschlußstutzen (17, 18) versehen ist, die in den Hohlraum münden und mit Anschlußstutzen der jeweils längs beabstandeten, in gleicher Winkelposition sich befindenden Grundkörper unter Ausbildung des Leitungsstücks (10) verbunden sind.

5. Endoskopschaft nach Anspruch 4, **dadurch gekennzeichnet, daß**
der Grundkörper an seinen Endflächen (8, 8') längs verlaufende Einkerbungen (7, 7') hat, die bei Bildung einer Schicht durch entsprechendes Aneinanderlegen zweier Körper (5) zwei diametrisch gegenüberliegende Durchgangskanäle (9, 9') bilden, durch die jeweils ein Leitungsstück (10) hindurch geführt ist.

## Claims

1. An endoscope shaft comprising a movable distal end portion (1) and an operating device which is operatively connected through the endoscope shaft to the distal end portion (1) for the operation thereof,
**characterized in that**
said distal end portion is composed of a plurality of longitudinally stacked swelling bodies (5), two of which, respectively, are located diametrically with respect to each other to form a layer, and two longitudinally directly adjacent pairs of bodies, respectively, are phase-shifted by 90°, wherein all swelling bodies (5) located at twelve, at three, at six and at nine o'clock, respectively, are longitudinally coupled with each other by means of a mechanical coupling which, respectively, is composed of at least one piece of duct (10) also forming the hydraulic or pneumatic connection at the same time, said duct extending between two swelling bodies, respectively, disposed diametrically to each other and being fixedly mounted to the swelling bodies to be coupled.

2. An endoscope shaft according to claim 1, **characterized in that**
each bellows has a semicircularly bent basic body, so that the two pairs of bodies of each layer interact to form a central through hole (12).

3. An endoscope shaft according to claim 2, **characterized in that** said basic body forms a hollow and is folded at its radial outside.

4. An endoscope shaft according to claim 3, **characterized in that** the basic body is provided, at its two front faces (15, 16), with connecting sleeves (17, 18) opening into the hollow and being connected to connecting sleeves of the respectively longitudinally spaced basic bodies disposed at an equal angular position while forming the piece of duct (10).

5. An endoscope shaft according to claim 4, **characterized in that** the basic body has, at its end faces (8, 8'), longitudinally extending notches (7, 7') which, upon the formation of a layer by appropriately juxtaposing two bodies (5), form two diametrically opposed through conduits (9, 9') through each of which a piece of conduit (10) is guided.

## Revendications

1. Tige endoscopique avec une section terminale distale (1) mobile ainsi qu'un dispositif d'actionnement qui est en liaison active, à travers la tige endoscopique, avec la section terminale distale (1) afin de l'actionner, **caractérisée en ce que** la section terminale distale (1) est constituée d'une pluralité de corps gonflables (5) empilés à cotés les uns des autres longitudinalement, dont chaque fois deux, diamétralement opposés, forment une couche et chaque fois deux paires de corps, immédiatement voisines sur la longueur, sont déphasées de 90°, tous les corps gonflables situés chaque fois à zéro, trois, six et neuf heures étant couplés entre eux longitudinalement au moyen d'un accouplement mécanique, qui est constitué chaque fois par au moins une portion de conduite (10) formant en même temps la liaison hydraulique ou pneumatique, qui s'étend chaque fois entre deux corps gonflables situés diamétralement en face l'un de l'autre et est fixé rigidement aux corps gonflables à accoupler.

2. Tige endoscopique selon la revendication 1, **caractérisée en ce que** chaque soufflet a un corps de base courbé en demi-cercle, de sorte que les deux paires de corps de chaque couche forment en coopérant une ouverture de passage centrale (12).

3. Tige endoscopique selon la revendication 2, **caractérisée en ce que** le corps de base forme un espace creux et est plissé sur sa face radialement extérieure.

4. Tige endoscopique selon la revendication 9, **caractérisée en ce que** le corps de base est pourvu sur ses deux faces frontales (15, 16) d'ajutages de raccordement (17, 18), qui débouchent dans l'espace creux et sont reliés à des ajutages de raccordement des corps de base, qui se trouvent chaque fois espacés longitudinalement, dans la même position angulaire en formant la portion de conduite (10).

5. Tige endoscopique selon la revendication 4, **caractérisée en ce que** le corps de base présente sur ses surfaces terminales (8, 8') des entailles (7, 7') s'étendant longitudinalement, qui réalisent, en formant une couche par disposition de deux corps (5) l'un sur l'autre, deux canaux de passage (9, 9') diamétralement opposés, par lesquels est guidé chaque fois une portion de conduite (10).
